# EUROPÄISCHE PATENTSCHRIFT

(11) **EP 1 915 114 B1**
(45) Veröffentlichungstag und Bekanntmachung des Hinweises auf die Patenterteilung: **18.11.2009**
(21) Anmeldenummer: 06778165.8
(22) Anmeldetag: 03.08.2006
(51) Int. Cl.: A61F 5/56, A61B 5/00

(54) **Vorrichtung zur Behandlung von Schnarchgeräuschen, Atemunterbrüchen und obstruktiver Schlafapnoe**
Device for treating snoring sounds, interruptions in breathing and obstructive sleep apnea
Dispositif pour traiter des ronflements, des interruptions de respiration et l'apnée du sommeil obstructive

(30) Priorität: 19.08.2005 CH 13642005
(43) Veröffentlichungstag der Anmeldung: 30.04.2008
(73) Patentinhaber: Velumount International GmbH, 3011 Bern (CH)
(72) Erfinder: WYSS, Arthur, CH-3011 Bern (CH)
(74) Vertreter: P&TS Patents & Technology Surveys SA
(86) Internationale Anmeldenummer: PCT/EP2006/065033
(87) Internationale Veröffentlichungsnummer: WO 2007/020197

(56) Entgegenhaltungen:
- DE-A1- 10 311 478
- DE-A1- 19 512 761
- FR-A- 2 838 046
- US-A- 4 669 459
- US-A- 5 490 520
- US-A- 6 089 864

## Beschreibung

### Technisches Gebiet

Die Erfindung bezieht sich auf eine Vorrichtung zur Behandlung von Schnarchgeräuschen, Atemunterbrüchen und obstruktiven Schlafapnoe (OSA) entsprechend dem unabhängigen Vorrichtungsanspruch, auf ein Verfahren zur Anpassung einer Vorrichtung an das Velum eines Patienten entsprechend dem Verfahrensanspruch 19 und auf ein Computerprogrammprodukt gemäss Anspruch 25.

### Stand der Technik

Schlafstörungen sind in der heutigen Gesellschaft aus verschiedenen Gründen weit verbreitet. Zu diesen Störungen gehört auch Schnarchen. Habituelles Schnarchen stellt ein soziales Problem dar, da es eine sehr störende Beeinträchtigung für den im gleichen Raum schlafenden Partner oder andere Personen sein kann. Habituelles Schnarchen ist für den Betroffenen nicht gesundheitsschädlich, ist aber bereits ein erstes Anzeichen für Verengungen beim Luftdurchfluss während des Schlafes. Mit zunehmendem Alter können diese Verengungen für den Betreffenden eine gesundheitliche Schädigung nach sich ziehen. Es kann sich ein Schlafapnoesyndrom entwickeln.

Es sind diverse Vorrichtungen zur Beeinflussung des Schnarchens und der obstruktiven Schlafapnoe im Stand der Technik bekannt. Diese Mittel haben sich jedoch mangels breiter Wirksamkeit praktisch nicht durchsetzen können.

Kieferprotrusionsschienen können Schnarchen und obstruktive Schlafapnoe lindern, können aber auch auf Dauer die Zahn- und Kieferstellung negativ beeinflussen. Zudem ist deren Anpassung von der Bezahnung abhängig.

Chirurgische Eingriffe können Linderung bringen, sind aber wie alle Operationen mit Risiken verbunden.

nCPAP-Geräte (nasale kontinuierliche positive Überdrucktherapie), die mit kontinuierlichem Überdruck während der Nacht die Luftwege offen halten und betroffenen Patienten das Atmen während des Schlafens ermöglichen, sind zur Zeit die Therapie der Wahl bei Schlafapnoe. Diese Geräte erfordern das Schlafen mit einer Maske, was wiederum für die Betroffenen soziale Probleme nach sich ziehen kann, da dies für den im gleichen Raum befindlichen Partner unter Umständen nicht angenehm ist. Zudem sind diese Geräte umfangreich und erfordern Wartungsaufwand und häufiger Verbrauchteilwechsel, was sich auf die allgemeinen Gesundheitskosten negativ auswirkt.

nCPAP-Geräte sind bei vielen Formen der Schlafapnoe wirksam, ungeachtet des Obstruktionsortes. Da sich viele, vor allem jüngere Patienten aber infolge oben genannter Gründe nicht mit einem nCPAP-Gerät abfinden können, wäre jedoch die genaue Lokalisation der Obstruktion wünschenswert, um diesen Personen weniger aufwändige und auch kostengünstigere Alternativen anbieten zu können.

Zur Evaluation von Schlafstörungen werden heute verschiedene Verfahren angewendet.

Pulsoxymeriegeräte, welche ambulant und stationär angewendet werden und welche die nächtliche Sauerstoffsättigung mittels einer am Finger angeschlossenen Elektrode messen, sind etabliert, zeigen aber nur bereits schwere Atemunterbrüche an. Habituelle Schnarcher welche erst geringe pathologische Atemmuster aufweisen, können damit nicht erfasst werden. Mit dieser Methode kann die Ursache einer Atemflussstörung nicht erkannt werden.

Atemmonitore, welche ambulant angewendet werden können, messen den Atemfluss bei den Naseneingängen. Mit dieser Methode kann die Ursache einer Atemflussstörung nicht erkannt werden. Atmet der Betroffene durch den Mund kann zudem das Atemsignal zu klein sein, um beim Naseneingang gemessen zu werden.

Die stationäre Schlafdiagnostik ist aufwändig und kostenintensiv. Dabei wird üblicherweise der Grad der Atemstörung genau diagnostiziert, nicht aber der exakte Ort der auslösenden Obstruktion.

Um den Ort der Obstruktion festzustellen, um damit dem Patienten eine spezifische Therapie zukommen lassen zu können, wird heute der Patient mittels eines schnell wirksamen Anästhetikums in Schlaf versetzt. Mittels Glasfaserkamera können dann Vibrationen und Obstruktionsorte im Pharynx eruiert werden. Diese Untersuchung ist aufwändig, invasiv und kostenintensiv. Zudem wird sie heute noch selten angewendet.

Die Plazierung von Vorrichtungen zu Therapie- und Diagnosezwecken direkt im Rachenraum war bisher ohne vorgängige Anästhesie des Patienten kaum möglich infolge des natürlichen Würgereflexes. Die Plazierung von Rohren durch den Mund im Pharynx zur Verhinderung von Schnarchen und Schlafapnoe wie in der Patentanmeldung WO-A1-98/09675 und DE-A1-195 01 363 vorgeschlagen oder die Platzierung eines Hohlkörpers mit Wandöffnungen durch die Nase wie in DE- A1-102 40 725 beschrieben, ist deshalb für einen Grossteil der Betroffenen nicht zumutbar.

In FR-A1-2,838,046 wird zudem ein flaches Dispositiv beschrieben, das vorne um die Schneidezähne gelegt und somit fixiert wird, flach in der Mitte zwischen Gaumen und Zunge liegt und hinter der Zunge gemittet, stufenförmig in den Rachen absteigt und an der Rachenhinterwand abstützt. Dieses Konzept kann wohl die Kollabierung der Zunge nach hinten verhindern, hat aber keinen Einfluss auf Obstruktionsorte im mittleren und oberen Pharynx. Das Schlucken wird durch die starke Abstützung an der hinteren unteren Pharynxwand stark erschwert.

US4669459 offenbart eine Anti-Schnarch-Vorrichtung, die darauf abzielt, Schwingungen im weichen Gaumen (soft palate) zu verhindern. Dazu wird von Mundinnenraum her ein leichter Druck ausgeübt.

Ein weitere Beispiel des Stands der Technik ist in FR 2 838 046 beschrieben.

### Darstellung der Erfindung

Aufgabe der Erfindung ist es, eine Vorrichtung eingangs genannter Art zu schaffen, die einfach herstellbar ist, verhältnismässig kostengünstig ist, und die sowohl zur Offenhaltung des Pharynx, zur Stabilisierung des Velums vom Pharynxraum her, zur Stabilisierung des Zungenrückens und Fernhaltung des Zungenrückens von der Pharynxhinterwand, zur Eruierung des Obstruktionsortes bei Schnarchern und Schlafapnoepatienten, zur Eruierung des Atemflusses im Pharynx, zum Training der Rachen- und Gaumenmuskulatur bei Schlaf-, Sprach- und Schluckstörungen dienen kann. Sie soll von der Bezahnung her unabhängig sein.

Damit sie durch den Mund im Pharynx bequem platzierbar ist, ohne einen übermässigen Würgereflex auszulösen, soll die Vorrichtung von aussen steuerbar sein.

Diese Aufgaben werden erfindungsgemäss durch die Merkmale der unabhängigen Ansprüche gelöst.

Im Speziellen werden die genannten Aufgaben durch eine Vorrichtung zur Behandlung von Schnarchgeräuschen, Atemunterbrüchen und obstruktiver Schlafapnoe (OSA) und zur Diagnose und Therapie von Obstruktionen und Vibrationen im Pharynxbereich gemäss Anspruch 1 gelöst.

Die Aufgaben werden auch durch ein Verfahren zur Anpassung einer erfindungsgemässen Vorrichtung an das Velum eines Patienten gemäss Anspruch 19 gelöst.

Die Aufgabe wird auch durch ein Computerprogrammprodukt, welches einen Softwarecode speichert gemäss Anspruch 25.

Vorteilhafte Ausführungsformen der vorliegenden Erfindung sind in den abhängigen Ansprüchen angegeben.

Die Mittel zur Stabilisierung des Velums können erfindungsgemäss aus einem oder einem doppelten Pharynxbogen, einem feinen Geflecht, einer Platte und/oder einer aufblasbaren oder mit einem Medium befüllbaren Form bestehen.

Die Oraltraversen können gerade oder rund geformt sein oder eine U-Form aufweisen und mit Referenzschenkeln an den Mundwinkeln befestigt werden oder direkt im Mund neben den Zahnreihen zusammengeführt werden, oder an einer für diese Anwendung gefertigte Zahnspange befestigt werden.

Die Mittel zur Stabilisierung des Velums sind an die Anatomie des Patienten anpassbar und können verschiedene Formen und Höhen aufweisen, so dass sie harmonisch an das Velum des Patienten anliegend geformt sind. Denkbar sind eine ∩-Form oder eine Ω-Form mit einem variablen Radius, eine M-Form, eine umgekehrte V-Form, eine Herz-Form, eine Ω-Form mit einem zusätzlichen oberen M oder andere Formen, die für denselben Zweck dienlich sind. Sie können nach hinten umgebogen sein, damit die Rachenwand zusätzlich abstützbar ist. Vorteilhaft kann ein doppelter Pharynxbogen vorhanden sein, wobei jeder Pharynxbogen separat vom Ende der Oraltrasversen oder von Referenzschenkeln steuerbar ist bzw. gesteuert wird.

Mit einer Zungenabsenkung zwischen den Oraltraversen und den Mitteln zur Stabilisierung des Velums kann retrolingual eine zusätzliche Zungenfixierung erreicht werden. Diese ist besonders vorteilhaft für Personen, deren Schnarch- und/oder Apnoeproblematik durch das Zurückfallen der Zunge verursacht wird.

Zusätze können an der Vorrichtung vorhanden sein, die das Gewebe weiter hinauf neben dem Nasenseptum stabilisieren.

Die Erfindung kann weiter nicht nur einteilig, sondern auch mehrteilig ausgeführt werden. Möglich sind beispielsweise eingeschobene oder aufgesetzte Knirschschoner auf der Zahnauflagefläche, mehrteilige Konstruktionen indem die Velum-stabilisierenden Mittel, die Oraltraversen und die Referenzschenkel ganz oder teilweise aus anderem Material als der Rest der Konstruktion gefertigt sind.

Die Vorrichtung besteht in einer Form aus einem Plastikschlauch, der im Inneren einen flexiblen Draht enthält. Dabei kann auch die Verwendung von ganz oder teilweise spiralförmig gedrehtem Federdraht vorgesehen sein, um feine Nuancen in Härte und Flexibilität zu erreichen. Auch eine Konstruktion von einem Plastik ummantelten Luftzwischenraum ist denkbar.

In einer weiteren Ausführungsform der erfindungsgemässen Spange können Mittel wie Sensoren oder Sonden für Bewegungs-, Temperatur-, Druck-, Elektrogramm-, Kopfpositions- und Akustikmessungen eingebaut sein. Alternativ oder zusätzlich kann aber auch die Ummantelung perforiert werden, damit die Messinstrumente zur Messung von Atemfluss, Druckverhältnissen, Muskeltätigkeit und Lage des Kopfes im Pharynx und zur Messung von Geräuschen an die Vorrichtung angeschlossen werden können. Die Sensoren können prinzipiell an den Mitteln zur Stabilisierung des Velums, am vorderen Ende der Oraltraversen oder an den Referenzschenkeln angebracht sein. Zu diesem Zweck sind elektrische Anschlüsse an den Referenzschenkeln (oder am vorderen Ende der Oraltraversen) vorhanden, die mit den Sensoren verbunden sind. Externe Geräte nehmen die Auswertung der gemessenen Daten auf bekannte Weise vor.

Eine weitere Ausführungsform für therapeutische Zwecke betrifft eine Einrichtung zum Training der Rachen- und Gaumenmuskulatur bei Schlaf-, Sprach- und Schluckstörungen. Am Pharynxbogen oder an den anderen genannten Mitteln zur Stabilisierung des Velums wird ein Ballon angebracht, der von aussen kurzfristig mit Luft, oder mit Flüssigkeit gefüllt werden kann und damit mit Schluckbewegungen ein Gaumenmuskulaturtraining ermöglicht.

Bei der hier beschriebenen Erfindung wird das Problem von Schnarchen und Schlafapnoe direkt am Entstehungsort im Pharynx und am Velum angegangen. Die Konstruktion ist einfach herzustellen, ist kostengünstig und verursacht auch kaum Brechreiz bei den Patienten. Sie ist einfach in den Mund einzuführen, sobald die Spange von einer Fachperson an die Anatomie des Patienten angepasst wurde. Danach kann der Patient die Spange ohne Probleme vor dem Schlafen anlegen und nach dem Aufstehen einfach herausnehmen. Diese Spange hat mit den beschriebenen einfachen Mitteln sehr gute Resultate bei Tests erzeugt. Auch die Reinigung ist sehr einfach, da es sich um Plastik handelt, der sich gut abwischen und trocknen lässt. Messungen zum Beispiel in einem Schlaflabor können vorteilhaft direkt am Entstehungsort abgenommen werden, in dem die externen Geräte an die Spange angeschlossen werden.

### Kurze Beschreibung der Figuren

Die Erfindung wird anhand der beigefügten Figuren näher erläutert, wobei zeigen
- Fig. 1: eine erste Ausführungsform einer erfindungsgemässen Spange bzw. Vorrichtung;
- Fig. 2: eine Oberansicht der Anordnung einer erfindungsgemässen Spange im Mund, wobei die Oraltraversen seitlich an den Zähnen vorbeigeführt sind;
- Fig. 3: eine seitliche Ansicht der Anordnung einer erfindungsgemässen Spange im Mund mit einer Zungenabsenkung;
- Fig. 4: den Bewegungsablauf bei der Einführung der Spange in den Mund;
- Fig. 5.1-5.3: verschiedene Ausführungsformen, um den Pharynxbogen an die Anatomie des Patienten anzupassen;
- Fig. 6: eine Ausführungsform einer erfindungsgemässen Spange mit einem doppelten Pharynxbogen;
- Fig. 7: eine Ausführungsform einer erfindungsgemässen Spange mit Dehnungsmesswiderständen im Pharynxbogen und elektrischen Anschlüssen;
- Fig. 8: eine Ausführungsform einer erfindungsgemässen Spange mit einer Perforierung mindestens im Pharynxbogen zur Anbringung verschiedener Sensoren, oder Sonden entweder direkt in der Perforation, am vorderen Ende der Oraltraversen oder an den Referenzschenkeln;
- Fig. 9: eine Ausführungsform einer erfindungsgemässen Spange mit einem Blasebalg und einem aufblasbaren Ballon;
- Fig. 10: einer schematischen Darstellung der Anwendung der Ausführungsform der Fig. 9 im Mund- und Rachenraum eines Patienten;
- Fig. 11: eine Ausführungsform einer erfindungsgemässen Spange mit aufgesetzten Knirschschonern;
- Fig. 12: eine Ausführungsform einer erfindungsgemässen Spange, die an einer Zahnplatte befestigt ist und
- Fig. 13: eine Ausführungsform einer erfindungsgemässen Vorrichtung mit einem feinem Gewebe oder einer Platte als Mittel zur Stabilisierung des Velums.

### Wege zur Ausführung der Erfindung

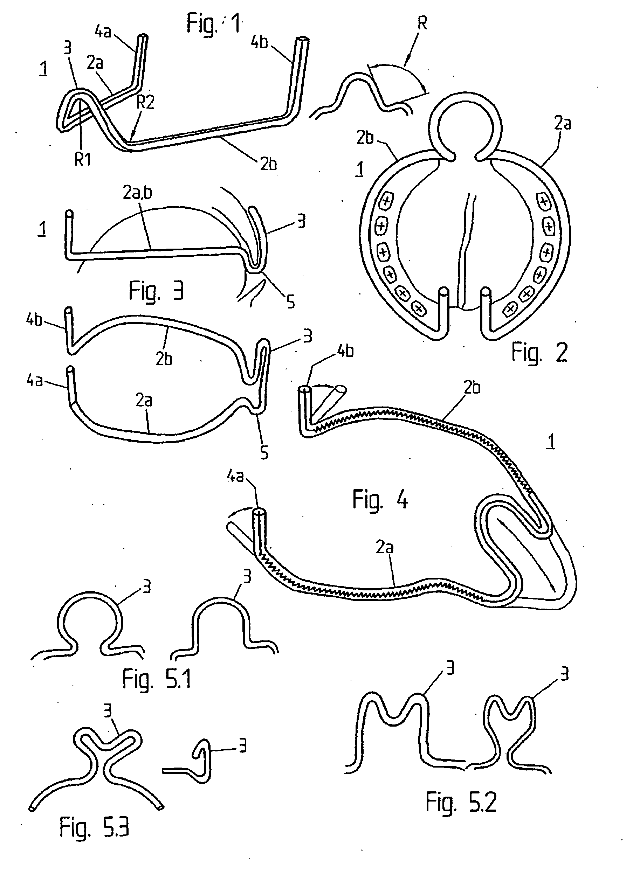

Eine erste Ausführungsform einer erfindungsgemässen Spange bzw. Vorrichtung 1 ist in der Fig. 1 sichtbar. Sie besteht aus zwei seitlichen Oraltraversen 2a, 2b und einem am hinteren Ende der Oraltraversen 2a, 2b angeordneten Pharynxbogen 3. Während der intraoralen Anwendung wird der Pharynxbogen 3 hinter dem Gaumensegel (Velum) plaziert und das Velum wird vom Rachen (Pharynx) her abgestützt und von der Rachenwand fernhalten, so dass die Atemwege durch die erfindungsgemässe Vorrichtung 1 während des Schlafs des Patienten freigehalten werden. Gleichzeitig wird das Bindegewebe im oberen Pharynx hinter dem Velum stabilisiert, so dass Obstruktionen verhindert werden können.

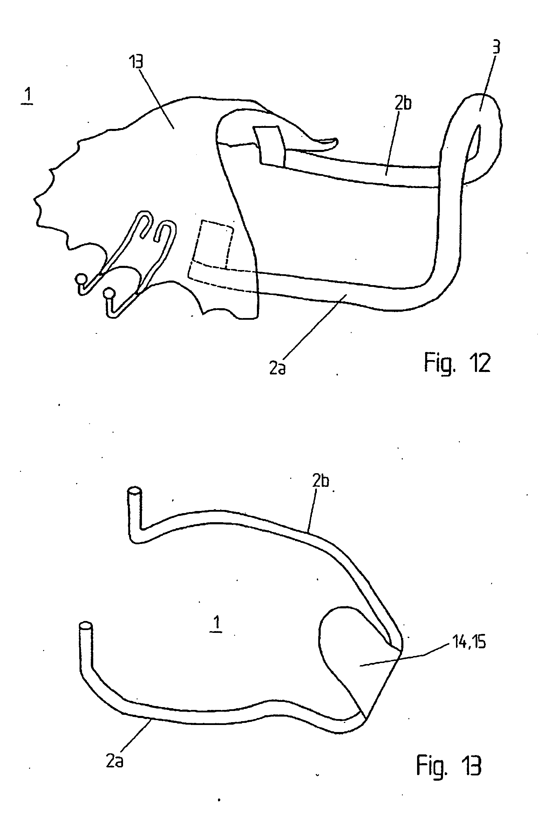

Als Mittel zur Stabilisierung des Velums können grundsätzlich in allen Ausführungsformen der erfindungsgemässen Vorrichtung 1 neben einem oder einem doppelten Pharynxbogen 3, ein feines Geflecht 14 (Fig. 13), eine Platte 15 (Fig. 13) und/oder eine aufblasbare oder befüllbare Form verwendet werden, wobei auch Kombinationen davon möglich sind. Eine Ausführungsform mit einem feinem Geflecht 13 oder einer Platte 14 ist in der Fig. 13 sichtbar. Andere Mittel, die denselben Zweck der Stabilisierung des Velums und des sich oberhalb des Velums befindende Bindegewebes erfüllen, sind aber im Rahmen der vorliegenden Erfindung ebenso einsetzbar.

Durch die erfindungsgemässe Vorrichtung 1 kommt es zu keinem Schnarchen und obstruktive Schlafapnoe kann massiv reduziert oder ganz verhindert werden. Mit zwei Referenzschenkeln 4a, 4b am vorderen Ende der Oraltraversen 2a, 2b wird die Vorrichtung 1 ausserhalb des Mundes fixiert. Die Fixierung kann aber auch durch andere geeignete Mittel geschehen und so ist vorstellbar, dass ein elastisches Band an den beiden Oraltraversen 2a, 2b befestigt ist, welches um den Kopf herum angeordnet ist. In einer anderen Ausführung werden die Oraltraversen 2a, 2b distal über den oberen Zahnreihen zusammengeführt und innerhalb des Mundes fixiert. Die Oraltraversen 2a, 2b können auch an einer Zahnplatte 13 innerhalb des Mundes befestigt sein, wie dies in der Ausführungsform der Fig. 12 sichtbar ist.

Die Oraltraversen 2a, 2b, die durch den Mund geführt werden, führen in geradem Weg von den Mundwinkeln über die seitlichen Zahnreihen zu den Arci palatopharyngae, wo die Oraltraversen 2a, 2b in einem Bogen mit einem Radius R₂ von 120° in dem umgekehrt V-förmigen oder ∩-förmigen Pharynxbogen 3 münden. Der Pharynxbogen 3 weist einen variablen Radius R₁ auf.

Die Vorrichtung 1 der Fig. 1 ist in einer einfachen Ausführungsform aus einer PVC-Draht-Konstruktion hergestellt und direkt über die Zähne und Zunge geführt. Dabei besteht sie in ihrer einfachsten Form aus einem Plastikschlauch, der im Inneren einen flexiblen Draht enthält. Wie in der Fig. 4 ersichtlich kann in einer weiteren Ausführung die Verwendung von ganz oder teilweise spiralförmig gedrehtem Federdraht als Teil der formgebenden Konstruktion verwendet werden, um feine Nuancen in Härte und Flexibilität der Vorrichtung 1 zu erreichen und um die Anpassung der Vorrichtung 1 an die Anatomie des Patienten zu erleichtern. Auch eine Konstruktion von einem Plastik ummantelten Luftzwischenraum ist denkbar.

Die Vorrichtung 1 kann aber auch aus anderen körperverträglichen und flexiblen, sowie in erforderlichem Masse stabilen Materialien bestehen. Die formgebende Konstruktion aber auch ganz in Kunststoff eingegossen werden. Ebenso kann die Ummantelung der formgebenden Konstruktion ganz, oder teilweise mit einem fliessfähigen Material befüllt werden. Eine weitere Ausführung beinhaltet, dass die Vorrichtung, oder Teile davon, mit einem körperverträglichen, gleitfähigen Material beschichtet werden können um Druckstellen im Rachen vorzubeugen und die Verträglichkeit zu verbessern.

Fig. 2 zeigt die Ansicht der Anordnung einer erfindungsgemässen Spange im Mund von oben. In dieser Ausführungsform können die Oraltraversen 2a, 2b neben den Zahnreihen geführt werden. Sie werden deshalb in der dafür notwendigen U-Form hergestellt oder aber generell rund geführt. In einer anderen Ausführung werden die Oraltraversen 2a, 2b distal über den oberen Zahnreihen zusammengeführt und innerhalb des Mundes fixiert. Bei dieser Version kann die intraorale Fixierung die Aufgabe der erwähnten Referenzschenkel 4a, 4b übernehmen.

Fig. 3 zeigt eine seitliche Ansicht einer Ausführungsform einer erfindungsgemässen Spange 1 im Mund. Für Personen, deren Schnarch- und/oder Apnoeproblematik durch das Zurückfallen der Zunge verursacht oder verstärkt wird, kann in dieser Ausführungsform eine Zungenabsenkung 5 unter den Arci palatopharyngae gefertigt werden.

Fig. 4 zeigt den Bewegungsablauf bei der intraoralen Einführung der Spange 1. Damit die Konstruktion unter der Uvula durch im Pharynx platziert werden kann, ohne die für den Würgereflex besonders empfindlichen Stellen zu berühren, ist der Pharynxbogen 3 mittels der Referenzschenkel 4a, 4b steuerbar bzw. flachlegbar.

Der Pharynxbogen 3 ist in seiner einfachsten Form einem umgekehrten V ähnlich (vgl. Fig. 1). Um der individuellen Anatomie und Problematik einzelner Individuen gerecht zu werden sind aber auch Ausführungen als ∩-Form oder als Ω-Form (Fig. 5.1) mit einem variablen Radius R₁ (Fig. 1), als M-Form (Fig. 5.2) oder als umgekehrte V-Form oder Herz (v)-Form, eine Ω-Form mit einem zusätzlichen oberen M (Fig. 5.2) oder andere Formen denkbar. Beispielsweise kann der Pharynxbogen 3 auch nach hinten umgebogen sein, der damit auch die Rachenhinterwand stützt, was der Konstruktion zusätzlichen Halt verleiht (Fig. 5.3). Weiter können Zusätze vorgesehen sein, die das Gewebe weiter hinauf neben dem Nasenseptum stabilisieren.

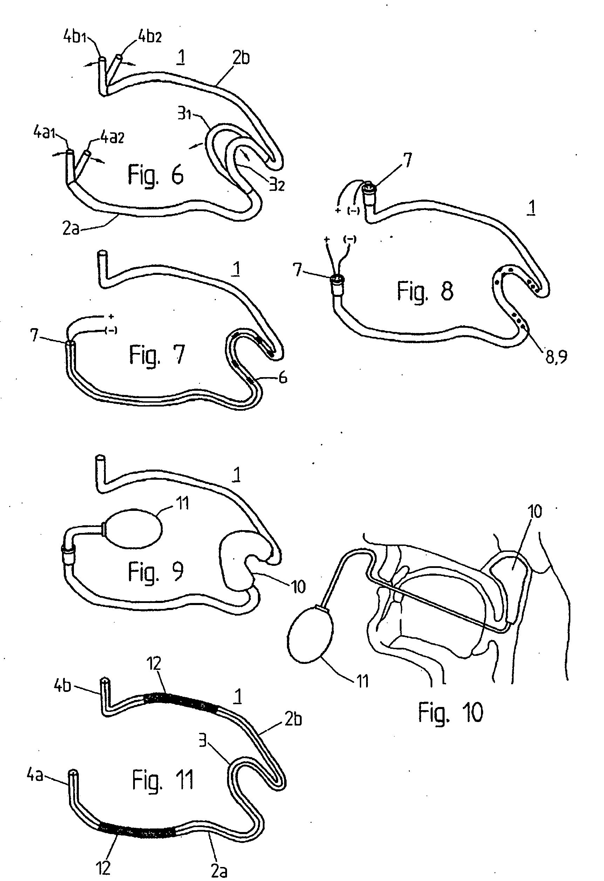

Fig. 6 zeigt eine Ausführungsform einer erfindungsgemässen Spange 1 mit doppeltem Pharynxbogen 3₁, 3₂, wobei ein Bogen 3₁ das Velum stabilisiert und der andere Bogen 3₂ an der Rachenhinterwand anzuliegen kommt. Dabei können die beiden Bögen 3₁, 3₂ auch unabhängig voneinander durch eine Doppelkonstruktion mit doppelten Referenzschenkeln 4a₁, 4b₁, 4a₂, 4b₂ gesteuert werden.

Wie in der Fig. 7 ersichtlich können im Pharynxbogen 3 in weiteren Ausführungsformen der erfindungsgemässen Spange 1 Mittel für spezifische Anwendungen wie Bewegungs-, Temperatur-, Druck-, Elektrogramm- (z.B. Elektromyogramm), Kopflage- und Akustikmessungen eingebaut sein. Alternativ oder zusätzlich wie in der Fig. 8 dargestellt kann aber auch die Ummantelung im Pharynxbogen 3 oder an den anderen genannten Mitteln zur Stabilisierung des Velums mit Perforationen 8 versehen werden, damit Messinstrumente (Sensoren 6, 9, Messsonden, etc.) zur Messung von Atemfluss, Druckverhältnissen im Pharynx und Geräuschen an die Vorrichtung angeschlossen werden können. Die Sensoren 9 können auch am vorderen Ende der Oraltraversen 2a, 2b und/oder an den Referenzschenkeln 4a, 4b angeordnet werden. Zum Zweck des Anschlusses der Geräte werden Anschlusskabel durch die Oraltraversen 2a, 2b geführt, die am Ende der Oraltraversen 2a, 2b oder an den Referenzschenkeln 4a, 4b in elektrische Anschlüsse 7 münden. An diese Anschlüsse 7 werden externe Geräte geschlossen (nicht in den Fig. dargestellt), die die Auswertung der gemessenen Daten auf bekannte Weise vornehmen. Selbstverständlich können die benannten Sensoren 6, 9 oder Sonden auch direkt am Pharynxbogen 3 an externe Geräte angeschlossen werden.

Eine weitere Ausführungsform für therapeutische Zwecke betrifft eine Einrichtung zum Training der Rachen- und Gaumenmuskulatur bei Schlaf-, Sprach- und Schluckstörungen. Entsprechend den Fig. 9 und 10 wird am Pharynxbogen 3 oder an den anderen genannten Mitteln zur Stabilisierung des Velums ein Ballon 10 angebracht, der von aussen kurzfristig mit Luft, oder mit Flüssigkeit gefüllt werden kann und damit mit Schluckbewegungen ein Gaumenmuskulaturtraining ermöglicht. Um den Ballon 10 von aussen zu aufzupumpen ist eine kleine Handpumpe bzw. ein Handblasebalg 11 vorhanden, der an einem Anschluss an einem Referenzschenkel 4a, 4b befestigt wird. Die Luft oder die Flüssigkeit kann dann durch die Oraltraversen 2a, 2b zum den Ballon 10 gelangen und diesen aufblasen.

Die Vorrichtung 1 kann weiter nicht nur einteilig, sondern auch mehrteilig ausgeführt werden. Möglich sind beispielsweise eingeschobene oder aufgesetzte Knirschschoner 12 auf der Zahnauflagefläche (Fig. 11) oder mehrteilige Konstruktionen indem Pharynxbogen 3 oder die anderen genannten Mittel zur Stabilisierung des Velums, Oraltraversen 2a, 2b und Referenzschenkel 4a, 4b ganz oder teilweise aus anderem Material als der Rest der Konstruktion gefertigt sind.

Zur Anpassung der Mittel 3 zur Stabilisierung des Velums können bildgebende Mittel wie beispielsweise eine minituriasierte Kamera intraoral und in den Pharynx eines Patienten eingeführt werden. Auf diese Weise werden Bilder des Velums des späteren Verwenders der Vorrichtung 1 aufgenommen, die auf einen Rechner übertragen werden. Mit Hilfe einer Software, die auf dem Rechner abläuft, wird aus den aufgenommenen Bildern die optimale Form der Mittel 3 zur Stabilisierung des Velums für die individuelle Person berechnet und dargestellt. Gleiches gilt auch für eine evtl. vorhandene Zungenabsenkung 5. Zur Herstellung einer erfindungsgemässen Vorrichtung 1 können dann handbetriebene oder automatische Geräte, wie Biegelehren, verwendet werden.

Entsprechend bezieht sich die vorliegende Erfindung auch auf ein Computerprogrammprodukt, welches einen Softwarecode speichert, welcher fähig ist, auf einem Rechner abzulaufen, um die entstandenen Bildern des Velums und des Pharynx des Patienten auszuwerten und die optimale Form der Mittel 3 zur Stabilisierung des Velums und/oder der Zungenabsenkung 5 zu berechnen und darzustellen.

Bei der hier beschriebenen Erfindung wird das Problem von Schnarchen und Schlafapnoe direkt am Entstehungsort im Pharynx und am Velum angegangen. Die Konstruktion ist einfach herzustellen, ist kostengünstig und verursacht auch entgegen bestehenden Meinungen keinen Brechreiz bei den Patienten. Sie ist einfach in den Mund einzuführen, sobald die Spange 1 an die Anatomie des Patienten von einer Fachperson angepasst wurde. Danach kann der Patient die Spange 1 ohne Probleme vor dem Schlafen anlegen und nach dem Aufstehen herausnehmen. Diese Spange hat mit den beschriebenen einfachen Mitteln sehr gute Resultate erzeugt. Auch die Reinigung ist sehr einfach, da es sich um Plastik handelt, der sich gut abwischen und trocknen lässt. Messungen zum Beispiel in einem Schlaflabor können vorteilhaft direkt am Entstehungsort abgenommen werden, in dem die externen Geräte an die Spange angeschlossen werden.

### Bezugszeichenliste

- 1: Vorrichtung, Spange
- 2a, 2b: Oraltraversen
- 3, 3₁, 3₂: Pharyxbogen
- 4a, 4b: Referenzschenkel
- 4a₁, 4b₁, 4a₂, 4b₂: Referenzschenkel
- 5: Zungenabsenkung
- 6: Messsensor
- 7: Anschlüsse
- 8: Perforation
- 9: Messsensor
- 10: Ballon
- 11: Handblasebalg
- 12: Knirschschoner
- 13: Zahnplatte
- 14: Feines Geflecht
- 15: Platte

- R₁, R₂: Radius

## Patentansprüche

1. Vorrichtung (1) zur Behandlung von Schnarchgeräuschen, Atemunterbrüchen und obstruktiver Schlafapnoe (OSA) und zur Diagnose und Therapie von Obstruktionen und Vibrationen im Pharynxbereich, wobei die Vorrichtung (1) intraoral anwendbar ist,
die Vorrichtung (1) aus mindestens einer Oraltraversen (2a, 2b) besteht und an einem hinteren Ende der Oraltraversen (2a, 2b) Mittel (3) zur Stabilisierung des Velums vorhanden sind, wobei die Mittel (3, 10, 13, 14) individuell an die Anatomie des Patienten anpassbar sind **dadurch gekennzeichnet, dass** während der intraoralen Anwendung der Vorrichtung (1) die Mittel (3, 10, 13, 14) zur Stabilisierung des Velums hinter das Velum plazierbar sind und das Velum von der Rachenwand fernhalten und das Bindegewebe im oberen Pharynx oberhalb des Velums stabilisieren um Obstruktionen und Vibrationen zu verhindern.

2. Vorrichtung (1) nach Anspruch 1, **gekennzeichnet, durch** einen oder einen doppelten Pharynxbogen (3), ein feines Geflecht (13), eine Platte (14) und/oder einer aufblasbaren oder befüllbaren Form (10) als Mittel zur Stabilisierung des Velums.

3. Vorrichtung (1) nach Anspruch 1 oder 2, **dadurch gekennzeichnet, dass** die Vorrichtung (1) aus zwei seitlichen Oraltraversen (2a, 2b) besteht, wobei am hinteren Ende der Oraltraversen (2a, 2b) die Mittel (3) zur Stabilisierung des Velums vorhanden sind.

4. Vorrichtung (1) nach einem der Ansprüche 1 bis 3, **dadurch gekennzeichnet, dass** an vorderen Ende der Oraltraversen (2a, 2b) Referenzschenkel (4a, 4b) zur Fixierung der Vorrichtung (1) ausserhalb des Mundes in den Mundwinkel vorhanden sind.

5. Vorrichtung (1) nach einem der Ansprüche 1 bis 4, **dadurch gekennzeichnet, dass** Mittel (13) zur Fixierung der Oraltraversen (2a, 2b) innerhalb des Mundes vorhanden sind.

6. Vorrichtung (1) nach einem der Ansprüche 1 bis 5, **dadurch gekennzeichnet, dass** die Oraltraversen (2a, 2b) gerade oder rund geformt sind oder eine U-Form aufweisen und neben den Zahnreihen geführt werden können.

7. Vorrichtung (1) nach einem der Ansprüche 1 bis 6, **dadurch gekennzeichnet, dass** die Mittel zur Stabilisierung des Velums nach hinten umgebogen sind, damit die Rachenwand zusätzlich abstützbar ist.

8. Vorrichtung (1) nach einem der Ansprüche 1 bis 7, **dadurch gekennzeichnet, dass** die Vorrichtung zur Fixierung der Zunge retrolingual eine Zungenabsenkung (5) zwischen den Oraltraversen (2a, 2b) und den Mitteln (3, 10, 13, 14) zur Stabilisierung des Velums aufweist.

9. Vorrichtung (1) nach einem der Ansprüche 1 bis 8, **dadurch gekennzeichnet, dass** die Mittel (3, 10, 13, 14) zur Stabilisierung des Velums durch Manipulation des vorderen Endes der Oraltraversen (2a, 2b) oder von Referenzschenkel (4a, 4b) steuerbar ist und damit unter dem Velum und der Uvula durch vom Pharynx her platzierbar ist.

10. Vorrichtung (1) nach einem der Ansprüche 1 bis 9, **dadurch gekennzeichnet, dass** die Oraltraversen (2a, 2b) Knirschschoner (12) aufweisen.

11. Vorrichtung (1) nach einem der Ansprüche 1 bis 10, **dadurch gekennzeichnet, dass** Zusätze an der Vorrichtung (1) vorhanden sind, die das Gewebe weiter hinauf als das Velum neben dem Nasenseptum stabilisieren.

12. Vorrichtung (1) nach einem der Ansprüche 1 bis 11, **dadurch gekennzeichnet, dass** die Vorrichtung (1) Sensoren (6, 9) oder Sonden für die Aufnahme von Bewegungs-, Temperatur-, Druck-, Atemfluss-, Elektrogramm-, Kopflage- und/oder Akkustikmessungen aufweisen.

13. Vorrichtung (1) nach einem der Ansprüche 1 bis 12, **dadurch gekennzeichnet, dass** die Vorrichtung (1) eine Ummantelung aufweist und mindestens die Ummantelung Perforationen (8) aufweist und dort Sensoren (9) oder Sonden zur Aufnahme von Temperatur Druck, Atemfluss, Elektrogramm, Kopflage und/oder Geräuschen vorhanden sind oder diese am vorderen Ende der Oraltraversen (2a, 2b) oder an den Referenzschenkeln (4a, 4b) angebracht sind.

14. Vorrichtung (1) nach Anspruch 12 oder 13, **dadurch gekennzeichnet, dass** die Sensoren (6, 9) oder Sonden an externe Geräte anschliessbar sind und die Verbindungen zwischen den benannten Sensoren (6) oder Sonden und den Anschlüssen (7) innerhalb der Vorrichtung (1) verlaufen.

15. Vorrichtung (1) nach einem der Ansprüche 1 bis 14, **dadurch gekennzeichnet, dass** die Vorrichtung (1) aus körperverträglichen und flexiblen Materialien besteht.

16. Vorrichtung (1) nach einem der Ansprüche 1 bis 15, **dadurch gekennzeichnet, dass** die Vorrichtung (1) ein- oder mehrteilig ausgeführt ist.

17. Vorrichtung (1) nach einem der Ansprüche 1 bis 16, **dadurch gekennzeichnet, dass** die Vorrichtung (1) aus einem von einem Plastik ummantelten Luftzwischenraum, einem fliessfähigen Material, einem Draht oder aus ganz oder teilweise spiralförmig gedrehtem Federdraht besteht und/oder ganz aus einem Kunststoff hergestellt ist.

18. Vorrichtung (1) nach einem der Ansprüche 1 bis 17, **dadurch gekennzeichnet, dass** die Vorrichtung (1) mindestens teilweise mit einem körperverträglichen, gleitfähigen Material beschichtet ist.

19. Verfahren zur Anpassung einer Vorrichtung (1) nach einem der vorangegangenen Ansprüche an das Velum eines Patienten, wobei die hinter das Velum plazierbaren Mittel (3, 10, 13, 14) zur Stabilisierung des Velums in Form und Höhe an die anatomie des Velums des Patienten angepasst werden.

20. Verfahren nach Anspruch 19, **dadurch gekennzeichnet, dass** eine Zungenabsenkung (5) zwischen den Oraltraversen (2a, 2b) und den Mitteln (3, 10, 13, 14) zur Stabilisierung des Velums zur Fixierung der Zunge retrolingual vorhanden ist und die Zungenabsenkung (5) ebenfalls an die Anatomie des Patienten angepasst wird.

21. Verfahren nach Anspruch 19 oder 20, **dadurch gekennzeichnet, dass** bildgebende Mittel intraoral und in den Pharynx eingeführt werden und mit so entstandenen Bildern des Velums mit Hilfe einer Software die optimale Form der Mittel (3, 10, 13, 14) zur Stabilisierung des Velums und/oder der Zungenabsenkung (5) berechnet und dargestellt werden.

22. Verfahren nach einem der Ansprüche 19 bis 21, **dadurch gekennzeichnet, dass** die Mittel (3, 10, 13, 14) zur Stabilisierung des Velums durch Manipulation des vorderen Endes der Oraltraversen (2a, 2b) oder von Referenzschenkeln (4a, 4b) gesteuert werden und damit unter dem Velum und der Uvula durch in den Pharynx platziert werden.

23. Verfahren nach Anspruch 22, **dadurch gekennzeichnet, dass** ein doppelter Pharynxbogen (3₁, 3₂) vorhanden ist und jeder Pharynxbogen (3₁ 3₂) separat vom Ende der Oraltrasversen (2a, 2b) oder von Referenzschenkeln (4a₁, 4b₁, 4a₂, 4b₂) gesteuert wird.

24. Verfahren nach einem der Ansprüche 19 bis 23, **dadurch gekennzeichnet, dass** zur Herstellung einer Vorrichtung 1 und zur Anpassung der Mittel (3, 10, 13, 14) zur Stabilisierung des Velum handbetriebene oder automatische Geräte verwendet werden.

25. Computerprogrammprodukt, welches einen Softwarecode speichert, welcher fähig ist, auf einem Rechner abzulaufen, um ein Verfahren entsprechend Anspruch 20 durchzuführen und die entstandenen Bildern des Velums und des Pharynx eines Patienten auszuwerten und die optimale Form der hinter das Velum plazierbaren Mittel (3, 10, 13, 14) zur Stabilisierung des Velums zu berechnen und darzustellen.

## Claims

1. Device (1) for treating snoring sounds, interruptions in breathing and obstructive sleep apnea (OSA) and for diagnosing and treating obstructions and vibrations in the pharyngeal region, with the device (1) being used intra-orally, where the device (1) is comprised of at least one oral transverse (2a, 2b) and of means (3) which are placed at the rear end of the oral transverse (2a, 2b) for stabilizing the velum, with the means (3, 10, 13, 14) being individually adaptable to the patient's anatomy **characterized in that** where during intra-oral use of the device (1) the means to stabilize the velum are to be placed behind the velum and the velum is held at a distance from the pharyngeal wall and the connective tissue in the upper pharynx above the velum is stabilized in order to prevent obstructions and vibrations.

2. Device (1) according to claim 1, **characterized by** one or a double pharynx arch (3), a fine mesh (13), a plate (14) and/or an inflatable or fillable form (10) as means for stabilizing the velum.

3. Device (1) according to claim 1 or 2, **characterized in that** the device (1) consists of two lateral oral traverses (2a, 2b), wherein the means (3) for stabilizing the velum are provided at the rear end of the oral traverses (2a, 2b).

4. Device (1) according to any of claims 1 to 3, **characterized in that** at the front end of the oral traverses (2a, 2b) reference shanks (4a, 4b) are provided for fastening the device (1) outside the mouth at the corners of the mouth.

5. Device (1) according to any of the claims 1 to 4, **characterized in that** means (13) for fastening the oral traverses (2a, 2b) are provided inside the mouth.

6. Device (1) according to one of the claims 1 to 5, **characterized in that** the oral traverses (2a, 2b) are shaped straight or round or have a U-shape and can be arranged next the row of teeth.

7. Device (1) according to one of the claims 1 to 6, **characterized in that** the means for stabilizing the velum are bend backwards in order for it to also support the throat back wall.

8. Device (1) according to one of the claims 1 to 7, **characterized in that** the device for immobilizing the tongue retro-lingually has a tongue depressor (5) between the oral traverses (2a, 2b) and the means (3, 10, 13, 14) for stabilizing the velum.

9. Device (1) according to one of the claims 1 to 8, **characterized in that** the means (3, 10, 13, 14) for stabilizing the velum can be controlled by manipulating the front end of the oral traverses (2a, 2b) or the reference shanks (4a, 4b) and can thus be placed through under the velum and the uvula from the pharynx.

10. Device (1) according to one of the claims 1 to 9, **characterized in that** the oral traverses (2a, 2b) have gnashing protection (12).

11. Device (1) according to one of the claims 1 to 10, **characterized in that** additional means are present on the device (1) for stabilizing the tissues higher up than the velum next to the nose septum.

12. Device (1) according to one of the claims 1 to 11, **characterized in that** the device (1) has sensors (6, 9) or probes for recording measurements of movement, temperature, pressure, breath flux, electrogram, head position and/or acoustics.

13. Device (1) according to one of the claims 1 to 12, **characterized in that** the device (1) has a sheathing and at least the sheathing has perforations (8) and sensors (9) or probes for recording temperature, pressure, breath flux, electrogram, head position and/or noises are provided there or are affixed at the front end of the oral traverses (2a, 2b) or on the reference shanks (4a, 4b).

14. Device (1) according to claim 12 or 13, **characterized in that** the sensors (6, 9) or probes can be connected to external appliances and the connections between said sensors (6) or probes and the connections (7) pass inside the device (1).

15. Device (1) according to one of the claims 1 to 14, **characterized in that** the device (1) consists in well-tolerated and flexible materials.

16. Device (1) according to one of the claims 1 to 15, **characterized in that** the device (1) is made in one piece or of several parts.

17. Device (1) according to one of the claims 1 to 16, **characterized in that** the device (1) is made of an air gap sheathed in plastic, a material capable of flowing, a wire or a fully or partly spiral-shaped twisted spring wire and/or is made totally of plastic.

18. Device (1) according to one of the claims 1 to 17, **characterized in that** the device (1) is sheathed at least partly with a well-tolerated material capable of flowing.

19. Method for adapting a device (1) according to one of the preceding claims to the velum of a patient, where the means (3, 10, 13, 14) placeable behind the velum for stabilizing the velum can be adapted in shape and height to the anatomy of the patient's velum.

20. Method according to claim 19, **characterized in that** a tongue depressor (5) is provided between the oral traverses (2a, 2b) and the means (3, 10, 13, 14) for stabilizing the velum for immobilizing the tongue retro-lingually and the tongue depressor (5) is also adapted to the patient's anatomy.

21. Method according to claim 19 or 20, **characterized in that** image-producing means are inserted intra-orally and in the pharynx and **in that** with these generated images of the velum with the aid of a software the optimum shape of the means (3, 10, 13, 14) for stabilizing the velum and/or the tongue depressor (5) can be computed and represented.

22. Method according to one of the claims 19 to 21, **characterized in that** the means (3, 10, 13, 14) for stabilizing the velum can be controlled by manipulating the front end of the oral traverses (2a, 2b) or the reference shanks (4a, 4b) and can thus be placed through under the velum and the uvula in the pharynx.

23. Method according to claim 22, **characterized in that** a double pharynx arch (3₁, 3₂) is provided and each pharynx arch is controlled separately from the end of the oral traverses (2a, 2b) or from the reference shanks (4a₁, 4b₁, 4a₂, 4b₂).

24. Method according to one of the claims 19 to 23, **characterized in that** for making a device (1) and for adapting the means (3, 10, 13, 14) for stabilizing the velum, hand-operated or automatic devices are used.

25. Computer program product that stores a software code capable of running on a computer to execute a method according to claim 20 and evaluate the generated images of the velum and of the pharynx of a patient and calculate and then display the optimum shape of the means (3, 10, 13, 14) placeable behind the velum for stabilizing the velum.

## Revendications

1. Dispositif (1) pour traiter des ronflements, des interruptions de respiration et l'apnée obstructive du sommeil (OSA) et pour diagnostiquer et soigner les obstructions et les vibrations dans la région du pharynx, tel que le dispositif (1) est utilisé intra-oralement,
selon lequel
le dispositif (1) est composé d'au moins une traverse orale (2a, 2b) et de moyens (3) situés à l'embout arrière de la traverse orale (2a, 2b) de stabilisation du voile du palais, lesquels moyens (3, 10, 13, 14) sont adaptables individuellement à l'anatomie du patient, pendant l'utilisation intra-orale du dispositif (1) les moyens de stabilisation du voile du palais (3, 10, 13, 14) sont placés derrière le voile et maintiennent le voile à distance de la paroi postérieure du pharynx et stabilisent les tissus conjonctifs dans le pharynx supérieur au-dessus du voile afin d'empêcher les obstructions et les vibrations.

2. Dispositif (1) selon la revendication 1, **caractérisé par** une arche de pharynx simple ou double (3), une fine toile (13), une plaquette (14) et/ou une poche pouvant être gonflée ou remplie (10) comme dispositifs pour stabiliser le voile.

3. Dispositif (1) selon la revendication 1 ou 2, **caractérisé en ce que** le dispositif (1) comprend deux traverses orales latérales (2a, 2b), et **en ce que** les moyens (3) de stabilisation du voile sont situés à l'embout arrière de la traverse orale (2a, 2b).

4. Dispositif (1) selon l'une des revendications 1 à 3, **caractérisé en ce que** sur la partie avant des traverses orales (2a, 2b) des tiges de référence (4a, 4b) sont disposées dans les coins de la bouche pour maintenir le dispositif (1) en dehors de la bouche.

5. Dispositif (1) selon l'une des revendications 1 à 4, **caractérisé en ce que** les moyens (13) pour maintenir les traverses orales (2a, 2b) sont disposés à l'intérieur de la bouche.

6. Dispositif (1) selon l'une des revendications 1 à 5, **caractérisé en ce que** les traverses orales (2a, 2b) sont de forme droite ou ronde ou en forme de U et peuvent être placées auprès de la rangée de dents.

7. Dispositif (1) selon l'une des revendications 1 à 6, **caractérisé en ce que** les moyens de stabilisation du voile sont pliés vers l'arrière de manière à ce que la paroi postérieure du pharynx soit également maintenue.

8. Dispositif (1) selon l'une des revendications 1 à 7, **caractérisé en ce que** le dispositif pour immobiliser la langue de manière rétro-linguale présente un abaisse-langue (5) entre les traverses orales (2a, 2b) et les moyens (3, 10, 13, 14) de stabilisation du voile.

9. Dispositif (1) selon l'une des revendications 1 à 8, **caractérisé en ce que** les moyens (3, 10, 13, 14) de stabilisation du voile peuvent être contrôlés par manipulation de la partie avant des traverses orales (2a, 2b) ou des tiges de référence (4a, 4b) et peuvent ainsi être placés en-dessous du voile et de la luette du pharynx.

10. Dispositif (1) selon l'une des revendications 1 à 9, **caractérisé en ce que** les traverses orales (2a, 2b) présentent des protections contre le grincement des dents (12).

11. Dispositif (1) selon l'une des revendications 1 à 10, **caractérisé en ce que** des moyens supplémentaires sont présents sur le dispositif (1) pour stabiliser les tissus situés plus en hauteur du voile près de la cloison nasale.

12. Dispositif (1) selon l'une des revendications 1 à 11, **caractérisé en ce que** le dispositif (1) a des capteurs (6, 9) ou des sondes pour l'enregistrement de mesures de mouvement, température, pression, débit respiratoire, électrogramme, position de la tête et/ou acoustique.

13. Dispositif (1) selon l'une des revendications 1 à 12, **caractérisé en ce que** le dispositif (1) présente une gaine et au moins la gaine présente des perforations (8) et que des capteurs (9) ou des sondes pour l'enregistrement de mesures de mouvement, température, pression, débit respiratoire, électrogramme, position de la tête et/ou bruits y sont contenus ou sont fixés sur la partie avant des traverses orales (2a, 2b) ou sur les tiges de référence (4a, 4b).

14. Dispositif (1) selon l'une des revendications 12 ou 13, **caractérisé en ce que** les capteurs (6, 9) ou les sondes peuvent être connectés à des appareils extérieurs et que les liaisons entre lesdits capteurs (6) ou sondes et les connexions (7) passent au travers du dispositif (1).

15. Dispositif (1) selon l'une des revendications 1 à 14, **caractérisé en ce que** le dispositif (1) est constitué de matériaux bien tolérés et flexibles.

16. Dispositif (1) selon l'une des revendications 1 à 14, **caractérisé en ce que** le dispositif (1) est construit en une ou plusieurs pièces.

17. Dispositif (1) selon l'une des revendications 1 à 16, **caractérisé en ce que** le dispositif (1) est constitué d'un interstice d'air enrobé de plastique, d'un matériau écoulable, d'un fil métallique ou bien complètement ou en partie d'un fil ressort enroulé en forme de spirale et/ou est construit intégralement en plastique.

18. Dispositif (1) selon l'une des revendications 1 à 17, **caractérisé en ce que** le dispositif (1) est enrobé au moins partiellement d'un matériau bien toléré et capable de glisser.

19. Méthode pour adapter le dispositif (1) selon l'une des revendications précédentes au voile du palais d'un patient, selon lequel les moyens (3, 10, 13, 14) positionnables derrière le voile pour stabiliser le voile peuvent être adaptés en forme et hauteur à l'anatomie du voile du patient.

20. Méthode selon la revendication 18, **caractérisée en ce que** un abaisse-langue (5) est situé entre les traverses orales (2a, 2b) et les moyens (3, 10, 13, 14) de stabilisation le voile et immobiliser la langue de manière rétro-linguale et que l'abaisse-langue (5) est également adapté à l'anatomie du patient.

21. Méthode selon la revendication 19 ou 20, **caractérisée en ce que** des moyens pour produire une image sont insérés intra-oralement ou dans le pharynx et **en ce qu'**avec les images générées du voile et avec l'aide d'in logiciel la forme optimale des moyens (3, 10, 13, 14) de stabilisation du voile et/ou de l'abaisse-langue (5) peut être calculée et représentée.

22. Méthode selon l'une des revendications 19 à 21, **caractérisée en ce que** les moyens (3, 10, 13, 14) de stabilisation du voile peuvent être contrôlés en manipulant la partie avant des traverses orales (2a, 2b) ou les tiges de référence (4a, 4b) et peuvent ainsi être placés en-dessous du voile et de la luette du pharynx.

23. Méthode selon la revendication 22, **caractérisée en ce qu'**elle présente une arche de pharynx double (3₁, 3₂) et que chaque arche de pharynx est contrôlée séparément par les embouts des traverses orales (2a, 2b) ou des tiges de référence (4a₁, 4b₁, 4a₂, 4b₂).

24. Méthode selon l'une des revendications 19 à 23, **caractérisée en ce que** des outils manuels ou automatisés sont utilisés pour produire un dispositif (1) et pour adapter les moyens (3, 10, 13, 14) de stabilisation du voile.

25. Produit logiciel sur lequel un code logiciel est enregistré, lequel peut être lancé sur un ordinateur pour exécuter une méthode selon la revendication 20 et évaluer les images générées du voile et du pharynx d'un patient et pour calculer et représenter la forme optimale des moyens (3, 10, 13, 14) positionnables derrière le voile pour stabiliser le voile.
